# EUROPEAN PATENT SPECIFICATION

(11) **EP 3 556 295 B1**
(45) Date of publication and mention of the grant of the patent: **27.07.2022**
(21) Application number: 17880438.1
(22) Date of filing: 14.11.2017
(51) Int. Cl.: A61B 6/14, G01T 1/20

(54) **INTRAORAL SENSOR**
INTRAORALER SENSOR
CAPTEUR INTRABUCCAL

(30) Priority: 15.12.2016 JP 2016243263
(43) Date of publication of application: 23.10.2019
(73) Proprietor: Hamamatsu Photonics K.K., Hamamatsu-shi, Shizuoka 435-8558 (JP)
(72) Inventor: NAKAO Keisuke, Hamamatsu-shi Shizuoka 435-8558 (JP); MIYAGUCHI Kazuhisa, Hamamatsu-shi Shizuoka 435-8558 (JP)
(74) Representative: Hoffmann Eitle
(86) International application number: PCT/JP2017/040949
(87) International publication number: WO 2018/110184

(56) References cited:
- EP-A2- 2 594 202
- WO-A1-2016/115117
- JP-A- H10 282 243
- US-A1- 2002 021 786
- US-A1- 2014 367 578

## Description

### Technical Field

One aspect of the present invention relates to an intraoral sensor.

### Background Art

Conventionally, a dental intraoral sensor has been known. For example, in Patent Literature 1, a radiation imaging apparatus used as being mounted on a back of a tooth in an oral cavity is described as a fourth embodiment. The radiation imaging apparatus includes a scintillator, a fiber optical plate (FOP), a CMOS imaging element, a base, and the like.

A dental intraoral sensor is also disclosed in Patent Literature 2. Patent Literature 3 relates to an image pickup device having a plurality of photoelectric converter substrates carrying respective input/output terminal connected to the photoelectric converters. Patent Literature 4 discloses an intraoral imaging apparatus comprising a scintillator, a focusing arrangement, and a back-illuminated type image sensor.

### Citation List

### Patent Literature

Patent Literature 1: Japanese Unexamined Patent Publication No. 2003-66148
Patent Literature 2: Japanese Unexamined Patent Publication No. H5-130990
Patent Literature 3: US 2002/021786 A1. Patent Literature 4: WO 2016/115117 A1

### Summary of Invention

### Technical Problem

An intraoral sensor disclosed in Patent Literatures 1 and 2 is used and inserted into the oral cavity to transparently observe teeth and gums. Therefore, such an intraoral sensor is required to be miniaturized without sacrificing an effective area of a sensor. Furthermore, in such an intraoral sensor, a structure is required which is hardly damaged due to an external force applied to a sensor when being manufactured or used.

One aspect of the present invention is made to solve the problems, and an object of the present invention is to provide an intraoral sensor which is miniaturized as possible without sacrificing an effective area of a sensor and has a structure which is hardly damaged.

### Solution to Problem

An intraoral sensor according to one aspect of the present invention includes a scintillator including a first surface and a second surface, a fiber optical plate disposed on the second surface side of the scintillator, an image sensor disposed on an opposite side of the fiber optical plate from the scintillator, and a wiring board disposed on an opposite side of the image sensor from the fiber optical plate, in which, when viewed from a direction perpendicular to the first surface of the scintillator, an outline of the scintillator, an outline of the fiber optical plate, an outline of the image sensor, and an outline of the wiring board are substantially aligned, characterized in that the image sensor is a back-surface irradiation type image sensor, and the intraoral sensor further comprises a supporting substrate configured to support the image sensor between the image sensor and the wiring board.

In the intraoral sensor, since the outline of the scintillator, the outline of the fiber optical plate, the outline of the image sensor, and the outline of the wiring board as viewed from the direction perpendicular to the first surface of the scintillator are substantially aligned, an area occupied by a portion which does not contribute to imaging as viewed from the same direction can be made substantially zero. Therefore, it is possible to miniaturize the intraoral sensor as possible without sacrificing the effective area of the sensor. Furthermore, if there is a step between edges of layers of the scintillator, the fiber optical plate, the image sensor, and the wiring board, a stress concentrates on the step, and the concentration of the stress may be a cause of peeling between layers and breakage of each layer. However, since the step is minimized in one aspect of the present invention, the peeling between the layers and the breakage of each layer hardly occur. In addition, where there is a portion in the image sensor and the wiring board which is not covered with the fiber optical plate, radiation having high energy is applied to the portion, and deterioration is facilitated. However, in one aspect of the present invention, since almost entire surfaces of the image sensor and the wiring board are covered with the fiber optical plate, such deterioration can be prevented.

In the intraoral sensor according to one aspect of the present invention, the outline of the image sensor may be configured not to protrude from the outline of the fiber optical plate and the outline of the wiring board. In this case, in a case where the outline of the image sensor, the outline of the fiber optical plate, and the outline of the wiring board are different from each other due to a manufacturing error and the like, the image sensor which is most likely to be damaged can be protected.

According to the claimed invention, since an electrical output can be taken from an opposite surface of the image sensor from the fiber optical plate, the image sensor and the wiring board can be easily and electrically connected.

Because the supporting substrate is used, the image sensor can be thinned in a state of being supported by the supporting substrate and can be connected to the wiring board. Therefore, the back-surface irradiation type image sensor which needs to be thinned can be easily manufactured and handled.

In the intraoral sensor according to one aspect of the present invention, the scintillator may be a film-like scintillator. In this case, the scintillator can be easily attached by an arbitrary process.

In the intraoral sensor according to one aspect of the present invention, the image sensor and the wiring board may be connected to each other with a ball-like conductor. In this case, the electrical connection between the image sensor and the wiring board can be easily and reliably performed.

The intraoral sensor according to one aspect of the present invention may include an underfill layer between the image sensor and the wiring board. In this case, since a gap between the image sensor and the wiring board does not exist, mechanical strength of the intraoral sensor is improved.

The intraoral sensor according to one aspect of the present invention may include a connector which is provided on an opposite surface of the wiring board from the image sensor and electrically connects a cable. In this case, the cable can be led from the opposite surface of the wiring board from the image sensor. Therefore, the intraoral sensor can be more easily inserted into the oral cavity in comparison with a structure in which the connector is provided at an edge of the wiring board and the cable is led from the edge.

The intraoral sensor according to one aspect of the present invention may include a sealed container. The scintillator, the fiber optical plate, the image sensor, and the wiring board may be sealed in the sealed container. In this case, the intraoral sensor can be inserted into the oral cavity and used.

### Advantageous Effects of Invention

According to one aspect of the present invention, it is possible to provide an intraoral sensor which is miniaturized as possible without sacrificing an effective area of a sensor and has a structure which is hardly damaged.

### Brief Description of Drawings

FIG. 1 is a diagram of an intraoral sensor according to a first embodiment.
FIG. 2 is a schematic diagram of an intraoral sensor as an internal configuration of the intraoral sensor illustrated in FIG. 1. FIG. 2(a) is a top view, FIG. 2(b) is a cross-sectional diagram taken along a b-b line in FIG. 2(a), and FIG. 2(c) is a diagram of a modification of Fig. 2(a).
FIG. 3 is a diagram of a process for manufacturing an image sensor supported by a supporting substrate of the intraoral sensor according to the first embodiment.
FIG. 4 is a diagram of the process for manufacturing the intraoral sensor according to the first embodiment subsequent to the process illustrated in FIG. 3.
FIG. 5 is a diagram of an intraoral sensor according to a second embodiment.
FIG. 6 is a diagram of a part of a process for manufacturing the intraoral sensor according to the second embodiment.
FIG. 7 is a diagram of an intraoral sensor according to an example not covered by the claimed invention.
FIG. 8 is a diagram of a process for manufacturing an image sensor supported by a supporting substrate of the intraoral sensor according to the example of FIG. 7.
FIG. 9 is a diagram of a process for manufacturing the intraoral sensor according to the example of FIG. subsequent to the process illustrated in FIG. 8.

### Description of Embodiments

Hereinafter, embodiments according to one aspect of the present invention will be described with reference to the drawings. In the drawings, the same or corresponding parts are denoted with the same reference numeral. Furthermore, there is a case where dimensions and dimensional ratios of each unit in the drawings do not indicate actual dimensions and dimensional ratios.

### [First Embodiment]

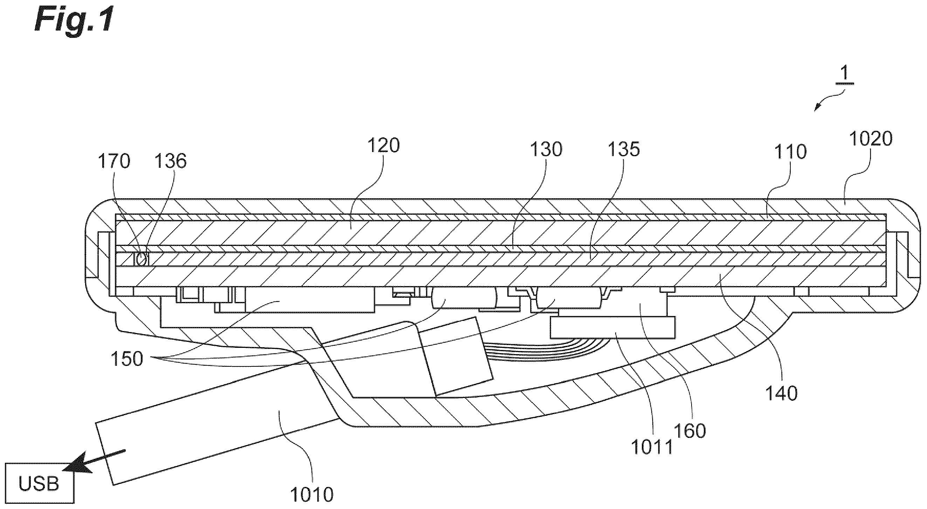

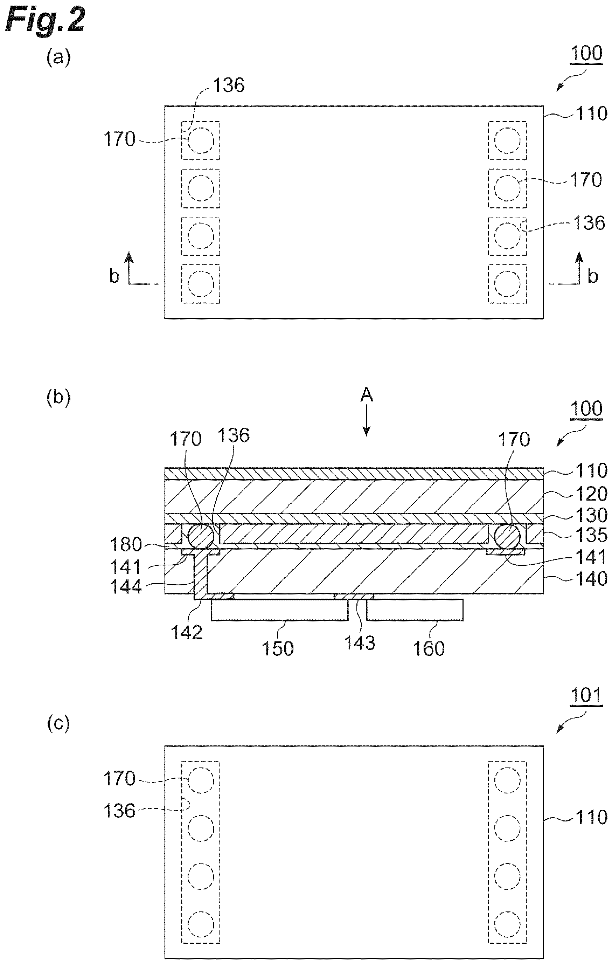

FIG. 1 illustrates an intraoral sensor 1 according to a first embodiment. FIGS. 2(a), 2(b), and 2(c) schematically illustrate intraoral sensors 100 and 101 as internal configurations of the intraoral sensor 1 illustrated in FIG. 1. As illustrated in FIGS. 1 and 2(a) to 2(c), the intraoral sensor 1 according to the first embodiment includes a scintillator 110, a fiber optical plate (FOP) 120, an image sensor 130, a wiring board 140, an IC 150, a connector 160, and a sealed container 1020. When viewed from a direction of an arrow A in FIG. 2(b) (direction perpendicular to first surface of scintillator), an outline of the scintillator 110, an outline of the FOP 120, an outline of the image sensor 130, and an outline of the wiring board 140 are substantially aligned.

Here, the outline of the scintillator 110, the outline of the FOP 120, the outline of the image sensor 130, and the outline of the wiring board 140 may be slightly different from each other due to a manufacturing error. However, the difference equal to or less than a thickness of the FOP can be allowed. Therefore, the case where the outlines are different from each other in a range equal to or less than the thickness of the FOP is included in the range of "substantially aligned". The difference can be equal to or less than half of the thickness of the FOP.

Furthermore, even when the difference is generated, the outlines of the image sensor 130 and a supporting substrate 135 do not protrude from the outlines of the FOP 120 and the wiring board 140. This can be realized by designing dimensions of the image sensor 130 and the supporting substrate 135 to be smaller than dimensions of other layers in consideration of tolerance.

The image sensor 130 is a so-called back-surface irradiation type image sensor, and a wiring layer is formed on the opposite side of a side where light enters (side of FOP 120). In an example, the thickness of the image sensor 130 is several µm to several tens µm. The image sensor 130 may be a CMOS type or a CCD type.

Furthermore, on the side of the image sensor 130 where the wiring layer is formed, the supporting substrate 135 which plays a role for supporting the image sensor 130 in a manufacturing process of the image sensor 130 is left. In an example, a material of the supporting substrate 135 is glass or Si.

An electrode of the image sensor 130 and an electrode 141 of the wiring board 140 are electrically connected with bumps (ball-like conductor) 170 disposed in through-holes 136 provided in the supporting substrate 135. A gap between the supporting substrate 135 and the wiring board 140 formed by the bumps 170 is filled with an underfill 180 to the end so as to increase mechanical strength of bump connection and make it difficult to apply an external force to the end. A material of the bump 170 is a solder and the like, and a material of the underfill 180 is a one-pack epoxy resin and the like.

In FIG. 2, the shape of the bump 170 is illustrated as a ball. However, it is not necessary for the shape of the bump 170 to be a ball, and any shape can be used. In FIG. 2(a), it is illustrated that the single through-hole 136 houses the single bump 170. However, as illustrated in FIG. 2(c), the single through-hole 136 may house the plurality bumps 170.

The scintillator 110 is a member which emits fluorescence (scintillation light) by receiving radiation. This may be manufactured integrally with the FOP 120 as a fiber optical plate with scintillator (FOS). A method for connecting the scintillator 110 with the FOP 120 includes a method for directly depositing the scintillator 110 on the FOP 120, a method for placing a film-like scintillator 110 on the FOP 120, and a method for bonding the scintillator 110 to the FOP 120 with a transparent resin.

The FOP 120 is a plate formed by bundling and bonding a large number of optical fibers formed of glass including metal atoms and the like, and has functions for transmitting light output from the scintillator 110 to the image sensor 130 and shielding radiation which has not been converted by the scintillator by the metal atoms (radiation transmitted through scintillator 110). In an example, the thickness of the FOP 120 is about 1.5 mm.

The wiring board 140 is a substrate on which the electrode 141, wirings 142 and 143, and the like are disposed and is, for example, a glass epoxy substrate (plate-like substrate obtained by impregnating epoxy resin in glass fiber and executing thermosetting treatment) or a ceramic substrate. In the wiring board 140, a through-hole 144 in which a conductive material such as solder is embedded is formed, and the electrode 141 and the wiring 142 are electrically connected to each other with the conductive material embedded in the through-hole 144. In an example, a thickness of the wiring board 140 is about 1 mm.

The IC 150 is an integrated circuit, for example, which plays roles as follows.
a. to supply a control clock signal to the image sensor 130.
b. to convert a supply voltage (for example, 5 V) from a cable into a voltage at which the image sensor 130 and other ICs (not shown) can operate.
c. to convert an image signal from the image sensor 130 into a format (for example, waveform conforming to USB standard) which can be transferred to an external device such as a personal computer.
d. to convert an instruction signal from a personal computer and the like into a format which can be transferred to the image sensor 130.
e. to hold information of the image sensor 130 and the like in a memory.

The connector 160 is a connector for connecting a cable (for example, USB cable 1010 illustrated in FIG. 1) which plays roles for deriving a signal from the IC 150 to the outside and supplying power or a signal from the outside to the IC 150 and the like.

As illustrated in FIG. 1, the intraoral sensors 100 and 101 configured as described above are connected to the USB cable 1010 and sealed in the sealed container 1020 and used in a form of the intraoral sensor 1.

The intraoral sensor 1 is inserted into an oral cavity of a patient and is disposed at a position which can be irradiated from a radiation source disposed outside the oral cavity and can detect radiation for transmitting teeth and gums. When the radiation is emitted from the radiation source, the intraoral sensors 100 and 101 in the intraoral sensor 1 detect the radiation which has transmitted the teeth and the gums and output signals of transparent images of the teeth and the gums.

According to the intraoral sensor 1, the intraoral sensor can be inserted into the oral cavity as it is and used.

Furthermore, in the intraoral sensors 1, 100, and 101, the outline of the scintillator 110, the outline of the FOP 120, the outline of the image sensor 130, and the outline of the wiring board 140 as viewed from the direction perpendicular to the first surface of the scintillator 110 (direction of arrow A) are substantially aligned. Therefore, an area occupied by a portion which does not contribute to imaging as viewed from the same direction can be made substantially zero. Therefore, it is possible to miniaturize the intraoral sensor as possible without sacrificing an effective area of the sensor. Furthermore, if there is a step between edges of each layers of the scintillator 110, the FOP 120, the image sensor 130, and the wiring board 140, the stress concentrates on the step, and the concentration of the stress may be a cause of peeling between layers and breakage of each layer. However, since the step is minimized in the intraoral sensors 1, 100, and 101, the peeling between the layers and the breakage of each layer hardly occur. In addition, when there is a portion in the image sensor 130 and the wiring board 140 which is not covered with the FOP 120, radiation having high energy is applied to the portion, and deterioration is facilitated. However, in the intraoral sensors 1, 100, and 101, since almost entire surfaces of the image sensor 130 and the wiring board 140 are covered with the FOP 120, such deterioration is prevented.

Furthermore, in the intraoral sensors 1, 100, and 101, the outline of the image sensor 130 is configured not to protrude from the outline of the FOP 120 and the outline of the wiring board 140. With this structure, even in a case where the outline of the image sensor 130, the outline of the FOP 120, and the outline of the wiring board 140 are slightly different from each other due to a manufacturing error and the like, the image sensor 130 which is most likely to be damaged is protected.

In the intraoral sensors 1, 100, and 101, it is assumed that the image sensor 130 be a back-surface irradiation type image sensor. By using the back-surface irradiation type image sensor, it is not necessary to provide an electrode on a light receiving surface of the image sensor 130, and the entire surface of the image sensor can be easily covered with the FOP. Furthermore, since an electrical output can be taken from an opposite side surface of the image sensor 130 from the FOP 120, the image sensor 130 and the wiring board 140 can be easily and electrically connected. In addition, sensitivity can be more improved in comparison with a case where a front-surface irradiation type image sensor is used.

Furthermore, in the intraoral sensors 1, 100, and 101, the supporting substrate 135 for supporting the image sensor is included between the image sensor 130 and the wiring board 140. When the supporting substrate 135 is used, since the image sensor 130 can be thinned as being supported by the supporting substrate 135 and can be connected to the wiring board 140, the back-surface irradiation type image sensor 130 which needs to be thinned can be easily manufactured and handled. Although the supporting substrate 135 is needed in a manufacturing process of the image sensor 130, there is no problem even if the supporting substrate 135 is left, and the mechanical strength can be maintained by leaving the supporting substrate 135. In a case where the fiber optical plate with scintillator (FOS) is used, the supporting substrate 135 may be left. This is because, although a scintillator portion of the FOS has a heat-sensitive property, a bonding process to the wiring board 140 in which the image sensor 130 is exposed to a high temperature can be completed in a state where the supporting substrate 135 is sandwiched between the image sensor 130 and the wiring board 140 before a process for bonding the FOS to the image sensor 130.

Furthermore, in the intraoral sensors 1, 100, and 101, the image sensor 130 and the wiring board 140 are electrically connected to each other with the bumps 170. With this structure, the electrical connection between the image sensor 130 and the wiring board 140 can be easily and reliably performed.

Furthermore, each of the intraoral sensors 1, 100, and 101 includes the layer of the underfill 180 between the supporting substrate 135 and the wiring board 140. With this structure, since the gap between the supporting substrate 135 and the wiring board 140 does not exist, the mechanical strength of each of the intraoral sensors 1, 100, and 101 is improved.

Furthermore, the intraoral sensors 1, 100, and 101 include the connector 160 which is provided on the opposite surface of the wiring board 140 from the image sensor 130 and electrically connects the cable. With this structure, since the cable can be led from the opposite surface of the wiring board 140 from the image sensor 130, the intraoral sensors 1, 100, and 101 can be more easily inserted into the oral cavity in comparison with a case where the connector 160 is provided at an edge of the wiring board 140 and the cable is led from the edge.

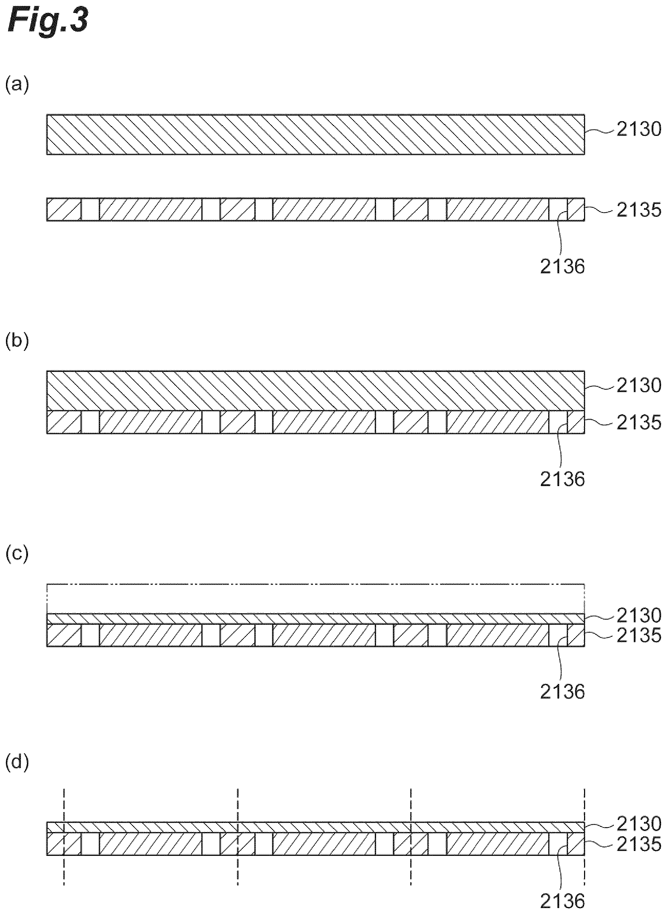

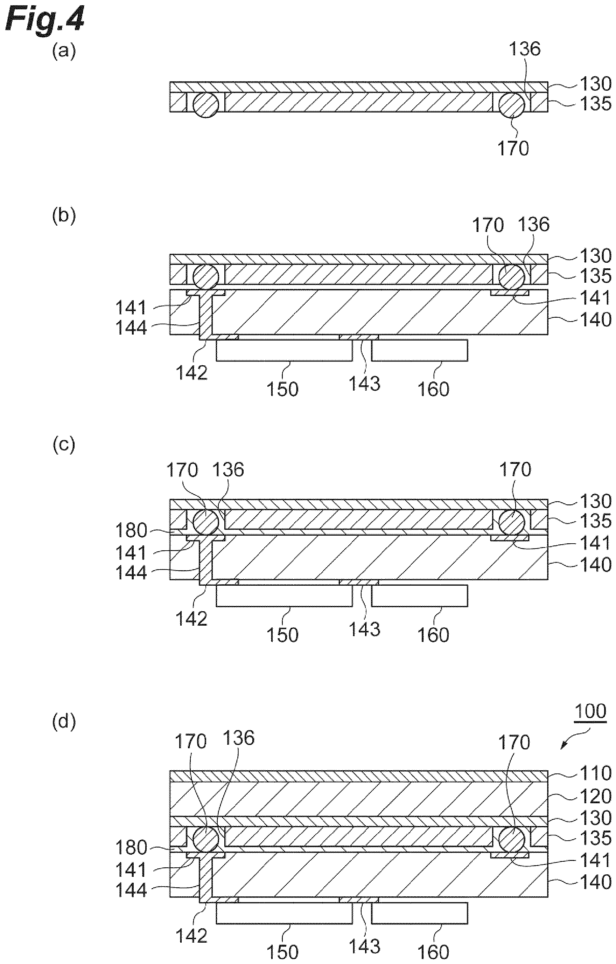

Next, an example of a manufacturing method of the intraoral sensors 1, 100, and 101 will be described with reference to FIGS. 3 and 4.

FIG. 3 is a diagram of a process for manufacturing the image sensor 130 supported by the supporting substrate 135, and FIG. 4 is a diagram of the process for manufacturing the intraoral sensors 1, 100, and 101 subsequent to the process illustrated in FIG. 3.

First, as illustrated in FIG. 3(a), a Si wafer 2130 in which elements configuring the plurality of image sensors 130 (impurity semiconductor region and electrode) are formed on a side of the first surface and a supporting substrate 2135 in which a through-hole 2136 is formed at a position corresponding to the position of each electrode of the plurality of image sensors 130 are prepared. Next, as illustrated in FIG. 3(b), the first surface of the Si wafer 2130 and the supporting substrate 2135 are positioned so that each electrode of each image sensor is exposed from the through-hole 2136 and are bonded to each other. Next, as illustrated in FIG. 3(c), a second surface side of the Si wafer 2130 is polished, and portions to be the plurality of image sensors 130 are thinned to have a predetermined thickness. Next, as illustrated in FIG. 3(d), the Si wafer 2130 is diced to be divided into the respective image sensors 130 supported by the supporting substrates 135. On the other hand, the wiring board 140 having the above structure is prepared in advance.

Next, as illustrated in FIG. 4(a), the bump 170 is inserted into the through-hole 136 of the supporting substrate 135. In a case where there is a possibility that electrical conduction with the bump 170 is prevented due to adhesion of adhesive to the surface of the electrode of the image sensor 130 exposed in the through-hole 136 and the like, an object which prevents the electrical conduction is removed by etching and the like before the bump 170 is inserted into the through-hole 136. Next, as illustrated in FIG. 4(b), the image sensor 130 supported by the supporting substrate 135 and the wiring board 140 are bonded with the bump 170, and in addition, the IC 150 and the connector 160 are mounted. The IC 150 and the connector 160 may be mounted before the bonding is performed or may be mounted after a process for filling the underfill 180 to be described next. Next, as illustrated in FIG. 4(c), a gap formed between the supporting substrate 135 and the wiring board 140 by the bump 170 is filled with the underfill 180. Next, as illustrated in FIG. 4(d), the FOP 120 and the scintillator 110 are bonded to the opposite side of the image sensor 130 from the supporting substrate 135 with a transparent resin. The FOP 120 and the scintillator 110 may be integrally manufactured as the fiber optical plate with scintillator (FOS) in advance as described above.

Finally, a connector 1011 of the USB cable 1010 is connected to the connector 160 of the intraoral sensors 100 and 101 and is sealed in the sealed container 1020 through which the USB cable 1010 passes, and the form illustrated in FIG. 1 is realized.

### [Second Embodiment]

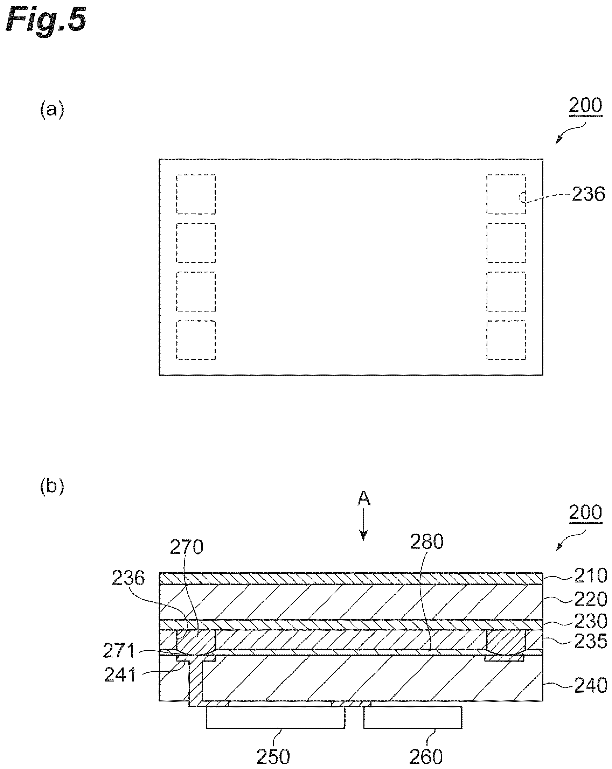

FIGS. 5(a) and 5(b) illustrate an intraoral sensor 200 according to a second embodiment.

As illustrated in FIGS. 5(a) and 5(b), the intraoral sensor 200 uses an embedded conductive member 270 instead of the bump 170 in the intraoral sensors 1, 100, and 101 according to the first embodiment. The intraoral sensor 200 is different from the intraoral sensors 1, 100, and 101 only in that point.

Hereinafter, a part of the intraoral sensor 200 different from the intraoral sensors 1, 100, and 101, that is, a part regarding the embedded conductive member 270 will be mainly described.

As illustrated in FIGS. 5(a) and 5(b), in the intraoral sensor 200, an electrode of an image sensor 230 and an electrode 241 of a wiring board 240 are embedded in a through-hole 236 provided in a supporting substrate 235, and the electrodes are electrically connected by the embedded conductive member 270 having a protruding portion 271. Furthermore, a gap between the supporting substrate 235 and the wiring board 240 formed by the protruding portion 271 of the embedded conductive member 270 is filled with an underfill 280 as in the intraoral sensors 1, 100, and 101. A material of the embedded conductive member 270 is a solder and the like.

The intraoral sensor 200 according to the second embodiment configured as described above has the same effect as the intraoral sensors 1, 100, and 101 according to the first embodiment.

In the first embodiment, regarding the through-hole 136, a modification illustrated in FIG. 2(c) may be employed. However, such a modification cannot be employed in the second embodiment. This is because insulation between the electrodes cannot be secured unless the through-hole is separated for each electrode in the second embodiment.

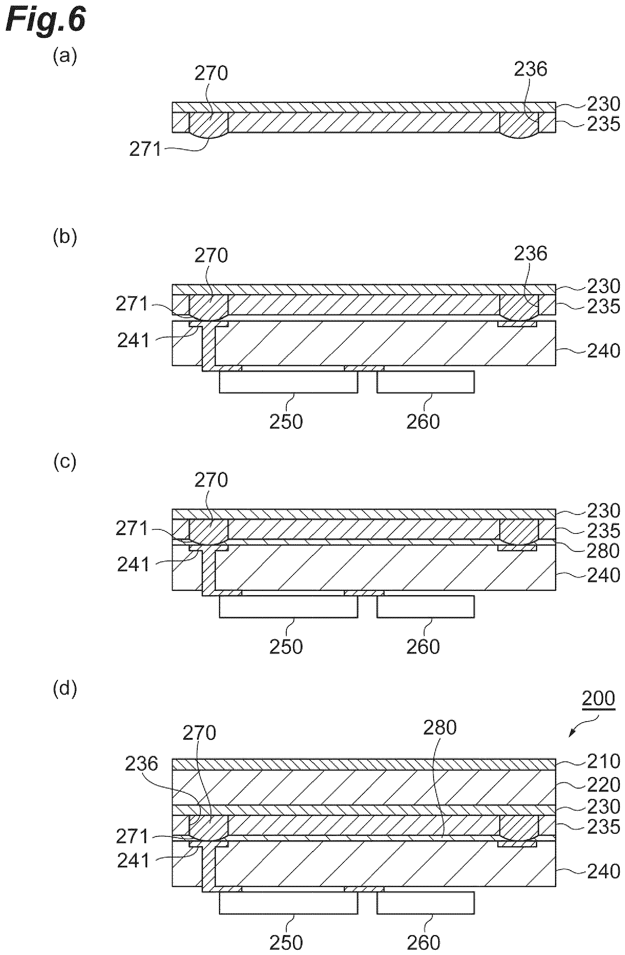

Next, an example of a manufacturing method of the intraoral sensor 200 described above will be described with reference to FIG. 6.

Note that a process for manufacturing the image sensor 230 supported by the supporting substrate 235 is the same as the process for manufacturing the image sensor 130 supported by the supporting substrate 135 illustrated in FIG. 3. Therefore, illustration and description of the process will be omitted.

FIG. 6 is a diagram of a process for manufacturing the intraoral sensor 200 from the image sensor 230 supported by the supporting substrate 235, in correspondence with FIG. 4 regarding the first embodiment.

Here, the wiring board 240 has the same configuration as the wiring board 140 in the first embodiment and is prepared in advance similar to the wiring board 140 in the first embodiment.

First, as illustrated in FIG. 6(a), the through-hole 236 of the supporting substrate 235 is filled with the embedded conductive member 270 so as to form the protruding portion 271. For example, this can be realized by a method for repeating introduction and reflow (melting of solder ball) of a solder ball into the through-hole 236 by a predetermined number of times. In a case where there is a possibility that electrical conduction with the embedded conductive member 270 is prevented due to adhesion of adhesive to the surface of the electrode of the image sensor 230 exposed in the through-hole 236 and the like, an object which prevents the electrical conduction is removed by etching and the like before the embedded conductive member 270 is filled into the through-hole 236. Next, as illustrated in FIG 6(b), the image sensor 230 supported by the supporting substrate 235 and the wiring board 240 are bonded to each other by the embedded conductive member 270 having the protruding portion 271, and in addition, an IC 250 and a connector 260 are mounted. The IC 250 and the connector 260 may be mounted before the bonding is performed or may be mounted after a process for filling the underfill 280 to be described next. Next, as illustrated in FIG. 6(c), a gap formed between the supporting substrate 235 and the wiring board 240 by the protruding portion 271 is filled with the underfill 280. Next, as illustrated in FIG. 6 (d), a FOP 220 and a scintillator 210 are bonded to the opposite side of the image sensor 230 from the supporting substrate 235. The FOP 220 and the scintillator 210 may be integrally manufactured as the fiber optical plate with scintillator (FOS) in advance as described above.

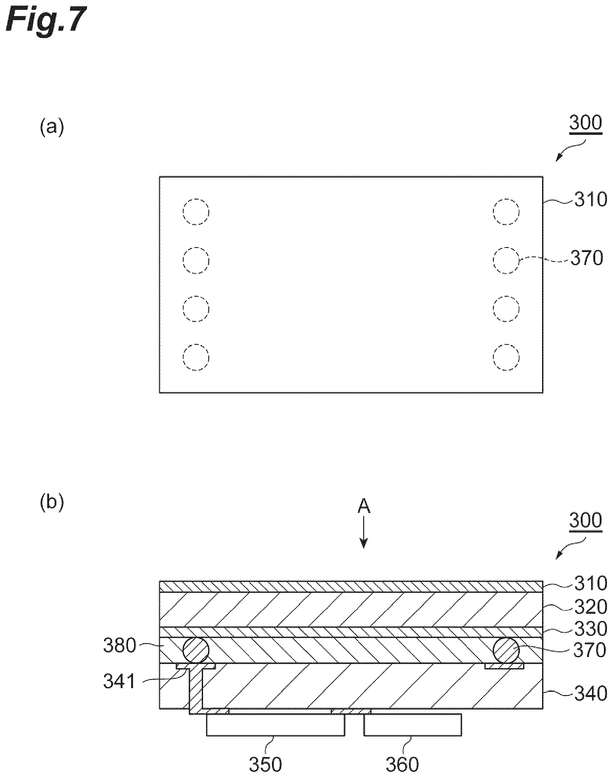

FIGS. 7(a) and 7(b) illustrate an intraoral sensor 300 according to an example not covered by the claimed invention.

As illustrated in FIGS. 7(a) and 7(b), the intraoral sensor 300 does not include the supporting substrate 135 of the intraoral sensors 1, 100, and 101 according to the first embodiment, and a scintillator 310 is a film-like scintillator. The intraoral sensor 300 is different from the intraoral sensors 1, 100, and 101 in only these two points.

Hereinafter, a part of the intraoral sensor 300 different from the intraoral sensors 1, 100, and 101 will be mainly described.

In the intraoral sensors 1, 100, and 101 according to the first embodiment, on the side of the image sensor 130 where the wiring layer is formed, the supporting substrate 135 which plays a role for supporting the image sensor 130 in the manufacturing process of the image sensor 130 is left. However, the supporting substrate is removed in the intraoral sensor 300 according to the present example of FIG.7, not covered by the claimed invention.

Then, an electrode of an image sensor 330 and an electrode 341 of a wiring board 340 are electrically connected with bumps (ball-like conductor) 370 as in the first embodiment. A gap between the image sensor 330 and the wiring board 340 formed by the bumps 370 is filled with an underfill 380. A material of the bump 370 is a solder and the like, and a material of the underfill 380 is a one-pack epoxy resin and the like.

In FIG. 7, the shape of the bump 370 is illustrated as a ball. However, it is not necessary for the shape of the bump 370 to be a ball, and any shape can be used.

The scintillator 310 is a film-like scintillator and is configured to be attachable to a FOP 320.

Note that, in the first embodiment, a fiber optical plate with scintillator (FOS) can be used as a scintillator. However, the scintillator 310 and the FOP 320 may be separated in the example of FIG.7, not covered by the claimed invention.

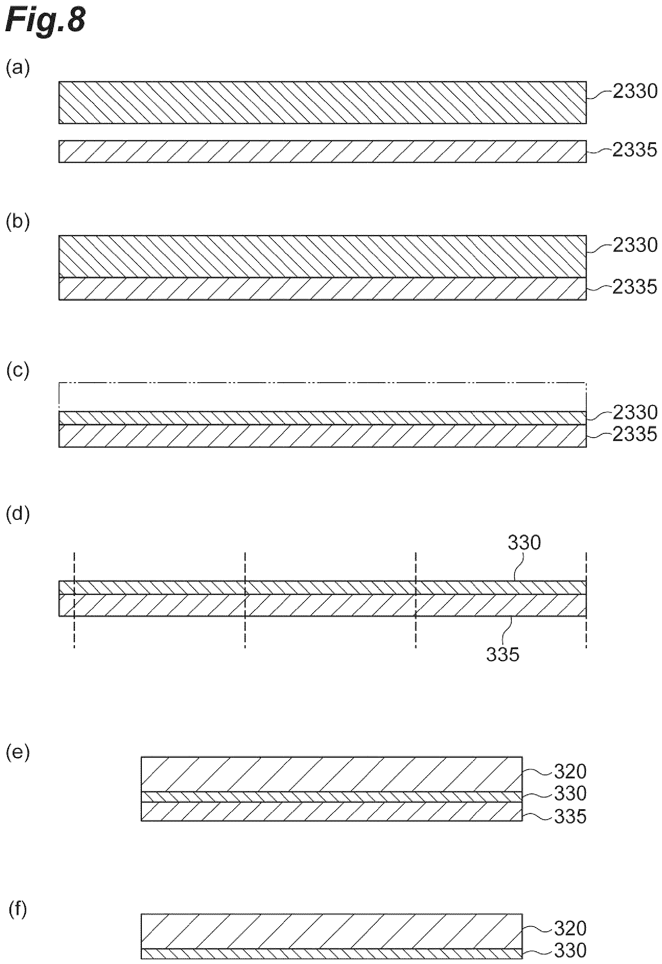

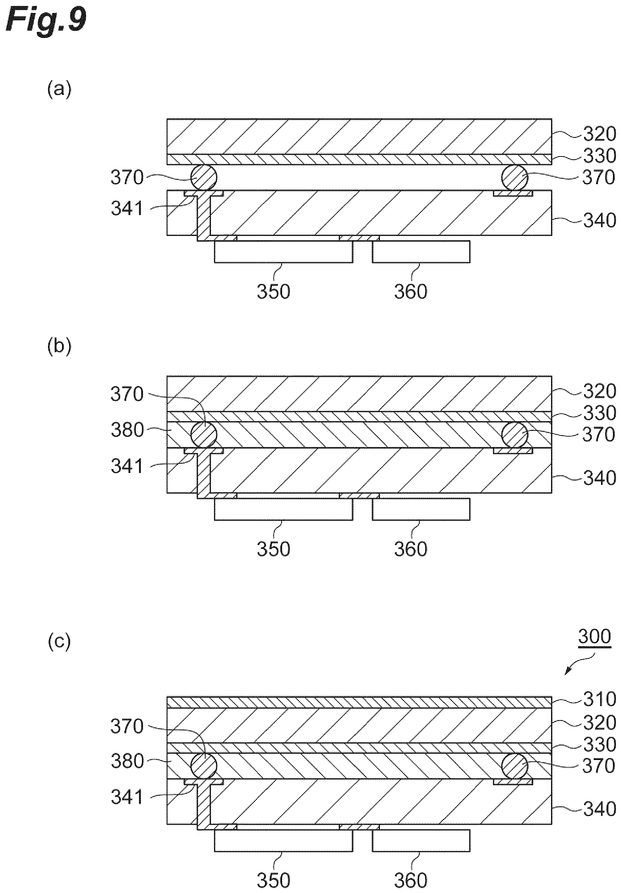

Next, an example of a manufacturing method of the intraoral sensor 300 described above will be described with reference to FIGS. 8 and 9.

FIG. 8 is a diagram of a process for manufacturing the image sensor 330 supported by the FOP 320, and FIG. 9 is a diagram of the process for manufacturing the intraoral sensor 300 subsequent to the process illustrated in FIG. 8.

First, as illustrated in FIG. 8(a), a Si wafer 2330 in which elements configuring the plurality of image sensors 330 (impurity semiconductor region and electrode) are formed on the side of the first surface and a supporting substrate 2335 are prepared. In FIG. 3 according to the first embodiment, the through-hole 2136 has been formed in the supporting substrate 2135. However, it is not necessary to provide a through-hole in the supporting substrate 2335 according to the present process of FIG. 8.

Next, as illustrated in FIG. 8(b), the first surface of the Si wafer 2330 is bonded to the supporting substrate 2335. Next, as illustrated in FIG. 8(c), a second surface side of the Si wafer 2330 is polished, and portions to be the plurality of image sensors 330 are thinned to have a predetermined thickness. Next, as illustrated in FIG. 8(d), the Si wafer 2330 is diced to be divided into the respective image sensors 330 supported by the supporting substrates 335. Next, as illustrated in FIG. 8(e), the FOP 320 is bonded on the opposite side of the image sensor 330 supported by the individual supporting substrate 335 from the supporting substrate 335. Next, as illustrated in FIG. 8(f), the supporting substrate 335 is removed. After the supporting substrate 335 has been removed, the thinned image sensor 330 is supported by the FOP 320. On the other hand, the wiring board 340 having the same structure as the wiring board 140 described above is prepared in advance.

Next, as illustrated in FIG. 9(a), the image sensor 330 and the wiring board 340 are bonded with the bumps 370, and in addition, an IC 350 and a connector 360 are mounted. The IC 350 and the connector 360 may be mounted before the bonding is performed or may be mounted after a process for filling the underfill 380 to be described next. Next, as illustrated in FIG. 9(b), a gap formed between the image sensor 330 and the wiring board 340 by the bumps 370 is filled with the underfill 380. Next, as illustrated in FIG. 9(c), the sheet-like scintillator 310 is bonded to the opposite side of the FOP 320 from the image sensor 330.

According to the manufacturing method illustrated in FIGS. 8 and 9, bonding with high temperature is performed after the image sensor 330 has been bonded to the FOP 320. Therefore, the FOP 320 needs to be able to withstand high temperature. However, since the scintillator 310 is bonded to the FOP 320 after the bonding, the scintillator 310 is not damaged by high temperature.

Although the embodiments according to one aspect of the present invention have been described above, the one aspect of the present invention is not limited to the embodiments, but rather by the appended claims. For example, in the embodiments, the shapes of the intraoral sensors 100 and 200 illustrated in the top view are rectangles. However, other shapes such as a polygon, a circle, and an ellipse may be used.

In the embodiments, a cross-sectional shape of each of the through-holes 136 and 236 of the supporting substrate is a square or a rectangle. However, other shapes such as a polygon, a circle, and an ellipse may be used.

In the intraoral sensors 100, 101, and 200 according to the embodiments, the ICs 150 and 250 are respectively provided in the intraoral sensors 100 and 200 one each. However, the plurality of ICs may be provided in each of the intraoral sensors 100, 101, and

In the embodiments, the cable is the USB cable 1010, and the connectors 160 and 260 are connectors connected to the USB cable 1010. However, the cable may be a cable other than the USB cable, and the connectors 160 and 260 may be a connector compatible with cables other than the USB cable.

In the embodiments, the image sensors 130 and 230 are back-surface incidence type image sensors.

### Industrial Applicability

It is possible to provide an intraoral sensor which is miniaturized as possible without sacrificing an effective area of a sensor and has a structure which is hardly damaged.

### Reference Signs List

- 1, 100, 101, 200, 300: intraoral sensor
- 110, 210, 310: scintillator
- 120, 220, 320: FOP
- 130, 230, 330: image sensor
- 135, 235, 335,2135, 2335: supporting substrate
- 136, 236, 2136: through-hole of supporting substrate
- 140, 240, 340: wiring board
- 141, 241, 341: electrode of wiring board
- 142, 143: wiring of wiring board
- 150,250,350: IC
- 160, 260, 360: connector (intraoral sensor side)
- 170, 370: bump
- 270: embedded conductive member
- 180, 280, 380: underfill
- 2130, 2330: Si wafer
- 1010: USB cable
- 1011: connector (USB cable side)
- 1020: sealed container

## Claims

1. An intraoral sensor (1, 100, 101) comprising:
a scintillator (110) including a first surface and a second surface;
a fiber optical plate (120) disposed on the second surface side of the scintillator (110);
an image sensor (130) disposed on an opposite side of the fiber optical plate (120) from the scintillator (110); and
a wiring board (140) disposed on an opposite side of the image sensor from the fiber optical plate (120),
wherein, when viewed from a direction perpendicular to the first surface of the scintillator (110), an outline of the scintillator (110), an outline of the fiber optical plate (120), an outline of the image sensor (130), and an outline of the wiring board (140) are substantially aligned,
**characterized in that**
the image sensor (130) is a back-surface irradiation type image sensor, and
the intraoral sensor (1, 100, 101) further comprises a supporting substrate (135) configured to support the image sensor (130) between the image sensor (130) and the wiring board (140).

2. The intraoral sensor (1, 100, 101) according to claim 1, wherein the outline of the image sensor (130) does not protrude from the outline of the fiber optical plate (120) and the outline of the wiring board (140).

3. The intraoral sensor (1, 100, 101) according to claim 1 or 2, wherein the scintillator (110) is a film-like scintillator.

4. The intraoral sensor (1, 100, 101) according to any one of claims 1 to 3, wherein the image sensor (130) and the wiring board (140) are connected to each other with a ball-like conductor.

5. The intraoral sensor (1, 100, 101) according to any one of claims 1 to 4, comprising: an underfill layer (180) between the image sensor (130) and the wiring board (140).

6. The intraoral sensor (1, 100, 101) according to any one of claims 1 to 5, comprising: a connector (160) configured to electrically connect a cable and be provided on an opposite surface of the wiring board (140) from the image sensor (130).

7. The intraoral sensor (1, 100, 101) according to any one of claims 1 to 6, further comprising: a sealed container (1020), wherein the scintillator (110), the fiber optical plate (120), the image sensor (130), and the wiring board (140) are sealed in the sealed container (1020).

## Patentansprüche

1. Intraoraler Sensor (1, 100, 101), umfassend:
einen Scintillator (110), der eine erste Fläche und eine zweite Fläche beinhaltet;
eine Glasfaserplatte (120), die an der zweiten Flächenseite des Scintillators (110) angeordnet ist;
einen Bildgebungssensor (130), der an einer dem Scintillator (110) gegenüberliegenden Seite der Glasfaserplatte (120) angeordnet ist; und
eine Verdrahtungsplatte (140), die an einer der Glasfaserplatte (120) gegenüberliegenden Seite des Bildgebungssensors angeordnet ist,
wobei, aus Sicht von einer Richtung senkrecht zur ersten Fläche des Scintillators (110), eine Kontur des Scintillators (110), eine Kontur der Glasfaserplatte (120), eine Kontur des Bildgebungssensors (130) und eine Kontur der Verdrahtungsplatte (140) im Wesentlichen miteinander ausgerichtet sind,
**dadurch gekennzeichnet, dass**
der Bildgebungssensor (130) ein Bildgebungssensor einer Rückflächenbestrahlungsart ist, und
der intraorale Sensor (1, 100, 101) weiter eine Stützunterlage (135) umfasst, die konfiguriert ist, um den Bildgebungssensor (130) zwischen dem Bildgebungssensor (130) und der Verdrahtungsplatte (140) zu stützen.

2. Intraoraler Sensor (1, 100, 101) nach Anspruch 1, wobei die Kontur des Bildgebungssensor (130) nicht von der Kontur der Glasfaserplatte (120) und der Kontur der Verdrahtungsplatte (140) hervorsteht.

3. Intraoraler Sensor (1, 100, 101) nach Anspruch 1 oder 2, wobei der Scintillator (110) ein filmähnlicher Scintillator ist.

4. Intraoraler Sensor (1, 100, 101) nach einem der Ansprüche 1 bis 3, wobei der Bildgebungssensor (130) und die Verdrahtungsplatte (140) mit einem kugelähnlichen Leiter miteinander verbunden sind.

5. Intraoraler Sensor (1, 100, 101) nach einem der Ansprüche 1 bis 4, umfassend: eine unausgefüllte Schicht (180) zwischen dem Bildgebungssensor (130) und der Verdrahtungsplatte (140).

6. Intraoraler Sensor (1, 100, 101) nach einem der Ansprüche 1 bis 5, umfassend: ein Verbindungsstück (160), das konfiguriert ist, um ein Kabel elektrisch anzuschließen und an einer dem Bildgebungssensor (130) gegenüberliegenden Fläche der Verdrahtungsplatte (140) bereitgestellt zu sein.

7. Intraoraler Sensor (1, 100, 101) nach einem der Ansprüche 1 bis 6, weiter umfassend: einen versiegelten Behälter (1020), wobei der Scintillator (110), die Glasfaserplatte (120), der Bildgebungssensor (130) und die Verdrahtungsplatte (140) in dem versiegelten Behälter (1020) versiegelt sind.

## Revendications

1. Capteur intra-oral (1, 100, 101) comprenant :
un scintillateur (110) incluant une première surface et une seconde surface ;
une plaque de fibres optiques (120) disposée sur le côté seconde surface du scintillateur (110) ;
un capteur d'image (130) disposé sur un côté opposé de la plaque de fibres optiques (120) par rapport au scintillateur (110) ; et
une carte de câblage (140) disposée sur un côté opposé du capteur d'image par rapport à la plaque de fibres optiques (120),
dans lequel, lorsqu'ils sont observés depuis une direction perpendiculaire à la première surface du scintillateur (110), un contour du scintillateur (110), un contour de la plaque de fibres optiques (120), un contour du capteur d'image (130), et un contour de la carte de câblage (140) sont sensiblement alignés,
**caractérisé en ce que**
le capteur d'image (130) est un capteur d'image de type rétroéclairé, et
le capteur intra-oral (1, 100, 101) comprend en outre une substrat de support (135) configuré pour supporter le capteur d'image (130) entre le capteur d'image (130) et la carte de câblage (140).

2. Capteur intra-oral (1, 100, 101) selon la revendication 1, dans lequel le contour du capteur d'image (130) ne fait ni saillie depuis le contour de la plaque de fibres optiques (120), ni depuis le contour de la carte de câblage (140).

3. Capteur intra-oral (1, 100, 101) selon la revendication 1 ou 2, dans lequel le scintillateur (110) est un scintillateur pelliculaire.

4. Capteur intra-oral (1, 100, 101) selon l'une quelconque des revendications 1 à 3, dans lequel le capteur d'image (130) et la carte de câblage (140) sont connectés l'un à l'autre avec un conducteur en forme de bille.

5. Capteur intra-oral (1, 100, 101) selon l'une quelconque des revendications 1 à 4, comprenant : une couche sous-jacente (180) entre le capteur d'image (130) et la carte de câblage (140).

6. Capteur intra-oral (1, 100, 101) selon l'une quelconque des revendications 1 à 5, comprenant : un connecteur (160) configuré pour connecter électriquement un câble et être fourni sur une surface opposée de la carte de câblage (140) par rapport au capteur d'image (130).

7. Capteur intra-oral (1, 100, 101) selon l'une quelconque des revendications 1 à 6, comprenant en outre : un contenant étanche (1020), dans lequel le scintillateur (110), la plaque de fibres optiques (120), le capteur d'image (130), et la carte de câblage (140) sont enfermés hermétiquement dans le contenant étanche (1020).
